# EUROPEAN PATENT APPLICATION

(11) **EP 4 365 187 A1**
(43) Date of publication of application: **08.05.2024**
(21) Application number: 22833570.9
(22) Date of filing: 28.06.2022
(51) Int. Cl.: C07K 7/06, C07K 7/08, C07K 14/00, A61K 38/00, A61P 35/00

(54) **PEPTIDE HAVING ANTICANCER ACTIVITY, AND USE THEREOF**

(30) Priority: 28.06.2021 KR 20210084285
(71) Applicant: Korea University Research and Business Foundation, Seoul 02841 (KR)
(72) Inventor: SHIN, Min Jeong, Seoul 06285 (KR); JEONG, In Hyeok, Goyang-si, Gyeonggi-do 10521 (KR)
(74) Representative: Maiwald GmbH
(86) International application number: PCT/KR2022/009207
(87) International publication number: WO 2023/277514

(57) **Abstract**

The present invention relates to a novel anticancer peptide. The anticancer peptide inhibits cancer cell proliferation and metastasis, induces cell death, and relieves drug resistance, and thus may be utilized for an anticancer use and an anticancer adjuvant.

## Description

### [Technical Field]

The present invention relates to a peptide having anticancer activity and a pharmaceutical composition for treating cancer, which includes the same.

### [Background Art]

Globally, the occurrence and mortality of cancer are increasing every year, and representative treatments include surgery, radiation, and chemotherapy. There are various types of cancer treatments, ranging from conventionally used cytotoxic anticancer agents to recently developed targeted anticancer drugs. Existing cytotoxic anticancer agents affect not only cancer cells but also normal cells, resulting in systemic toxicity and cytotoxicity, which causes various side effects. To overcome the limitations of cytotoxic anticancer agents, research and development on monoclonal antibody treatments that only target and kill cancer cells is being conducted, and there are already therapeutic agents approved by the US Food and Drug Administration (FDA). The approved monoclonal antibodies include cetuximab, avelumab, rituximab, trastuzumab, alemtuzumab, bevacizumab, and ipilimumab.

However, according to recent clinical results, it is known that the effect of some monoclonal antibodies is significantly reduced in patients with genetic mutations located downstream of a receptor in the intracellular signaling system. Therefore, the need for novel cancer therapeutic agents remains.

### [Disclosure]

### [Technical Problem]

Against the background, the present inventors discovered a novel functional peptide, and confirmed that this peptide inhibits the proliferation and metastasis of cancer cells, induces apoptosis, and improves drug resistance.

Accordingly, the present invention is directed to providing a peptide having anticancer activity and a pharmaceutical composition for preventing or treating cancer, which includes the same.

### [Technical Solution]

To achieve the above purpose, one aspect of the present invention provides a pharmaceutical composition for preventing or treating cancer, which includes a peptide ("anticancer peptide") that has an amino acid sequence selected from the group consisting of SEQ ID NOs: 3 to 5 as an active ingredient.

The term "prevention" used herein refers to all actions of inhibiting the progression of a disease or delaying the onset thereof by the administration of the pharmaceutical composition according to the present invention.

The term "treatment" used herein refers to all actions involved in alleviating or beneficially changing symptoms of a disease by the administration of the pharmaceutical composition according to the present invention.

In the present invention, the cancer may be a solid cancer, and specifically, selected from the group consisting of lung cancer, ovarian cancer, colorectal cancer, colon cancer, pancreatic cancer, liver cancer, cervical cancer, kidney cancer, stomach cancer, prostate cancer, breast cancer, brain tumors, uterine cancer, bladder cancer, but the present invention is not limited thereto.

According to one embodiment of the present invention, the peptide including the amino acid sequence represented by SEQ ID NO: 3 may be a peptide consisting of the amino acid sequence represented by SEQ ID NO: 1, 2, or 3. That is, the amino acid sequences of SEQ ID NOs: 1, 2, and 3 include the sequence of SEQ ID NO: 3 in common.

In addition, according to one embodiment of the present invention, the peptide including the amino acid sequence represented by SEQ ID NO: 4 or 5 may be a peptide consisting of the amino acid sequence represented by SEQ ID NO: 4 or 5.

According to one embodiment of the present invention, the pharmaceutical composition may include a peptide consisting of an amino acid sequence selected from the group consisting of SEQ ID NOs: 3 to 5, a peptide having 80% or more sequence homology with the amino acid sequence, or a peptide, which is a fragment thereof.

More specifically, the peptide disclosed herein may include a peptide having 80% or more, 85% or more, 90% or more, 95% or more, 96% or more, 97% or more, 98% or more, or 99% or more sequence homology. In addition, the peptide disclosed herein may include a peptide in which one or more, 2 or more, 3 or more, 4 or more, 5 or more, 6 or more, or 7 or more amino acids are changed from the peptide consisting of an amino acid sequence selected from the group consisting of SEQ ID NOs: 1 to 8, or fragments thereof.

In one embodiment, amino acid changes are properties that allow the physicochemical properties of a peptide to be changed. For example, amino acid changes, which improve the thermal stability, change substrate specificity, and change optimal pH, may be performed.

The "amino acid" used in the specification includes not only the 22 standard amino acids naturally incorporated into peptides, but also D-isomers and modified amino acids. Accordingly, in one aspect of the present invention, the peptide may be a peptide including a D-amino acid. Meanwhile, in another aspect of the present invention, the peptide may include a non-standard amino acid which has undergone post-translational modification. Examples of the post-translational modification include phosphorylation, glycosylation, acylation (e.g., including acetylation, myristoylation, and palmitoylation), alkylation, carboxylation, hydroxylation, glycation, biotinylation, ubiquitinylation, changes in chemical properties (e.g., β-elimination deimidization and deamination) and structural changes (e.g., formation of a disulfide bridge). In addition, the post-translational modification includes changes in amino acids caused by chemical reactions occurring in the process of bonding with crosslinkers for forming a peptide conjugate, such as changes in amino groups, carboxyl groups, or side chains.

The peptide disclosed in the specification may be a wild-type peptide that is identified and isolated from a natural source. Meanwhile, the peptide disclosed in the specification may be an artificial variant that includes an amino acid sequence in which one or more amino acids are substituted, deleted and/or inserted compared to a peptide consisting of an amino acid sequence selected from the group consisting of SEQ ID NOs: 1 to 8. Amino acid changes in wild-type polypeptides as well as in artificial variants include conservative amino acid substitutions that do not significantly affect the folding and/or activity of a protein. Conservative substitutions are made in groups of basic amino acids (arginine (Ala), lysine (Lys), and histidine (His)), acidic amino acids (glutamic acid (Glu) and aspartic acid (Asp)), polar amino acid (glutamine (Gln) and asparagine (Asp)), hydrophobic amino acids (leucine (Leu), isoleucine (Ile), and methionine (Met)), aromatic amino acids (phenylalanine (Phe), tryptophan (Trp), and tyrosine (Tyr)), and small amino acids (glycine (Gly), alanine (Ala), and threonine (Thr)). Amino acid substitutions that generally do not alter specific activity are known in the art. The most common exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, or vice versa.

Substantial modifications in the biological properties of a peptide are performed by selecting a substitution that differs significantly in (a) its effect in maintaining the structure of a polypeptide backbone, e.g., sheet or helical confirmation, in a substituted area, (b) its effect in maintaining the charge or hydrophobicity of the molecule in a target site, or (c) its effect in maintaining the bulk of a side chain. Natural residues are classified into the following groups based on common side chain properties:
(1) hydrophobic residues: norleucine (Nle), Met, Ala, valine (Val), Leu, Ile;
(2) neutral-hydrophilic residues: cysteine (Cys), serine (Ser), Thr;
(3) acidic residues: Asp, Glu;
(4) basic residues: asparagine (Asn), Gln, His, Lys, arginine (Arg);
(5) residues that affect chain orientation: Gly, proline (Pro); and
(6) aromatic residues: Trp, Tyr, Phe.

Non-conservative substitutions may be made by exchanging a member of one of these classes with a member of another class. Any cysteine residue that is not involved in maintaining the proper conformation of a peptide may usually be substituted with serine to improve the oxidative stability of the molecule and prevent unusual crosslinking. Conversely, cysteine bond(s) may be added to the peptide to improve its stability.

Another type of amino acid variant in peptides has an altered glycosylation pattern. The change refers to the deletion of one or more carbohydrate moieties found in a peptide and/or the addition of one or more glycosylation sites not present in a peptide.

The glycosylation of a peptide is typically N-linked or O-linked. "N-linked" means a carbohydrate moiety attached to a side chain of an asparagine residue. Tripeptide sequences, asparagine-X-serine and asparagine-X-threonine (herein, X is any amino acid except proline), are recognition sequences for enzymatic attachment of a hydrocarbon moiety to an asparagine side chain. Accordingly, since one of these tripeptide sequences is present in a polypeptide, a potential glycosylation site is created. O-linked glycosylation means the attachment of one of sugars, N-acetyl galactosamine, galactose, or xylose, to a hydroxyl amino acid, most commonly serine or threonine. Alternatively, 5-hydroxyproline or 5-hydroxylysine may also be used.

The addition of a glycosylation site to a peptide is conveniently made by changing the amino acid sequence to contain one or more tripeptide sequences, which are mentioned above (in the case of an N-linked glycosylation site). Such a change may be made by adding one or more serine or threonine residues to the sequence of the initial antibody or substitution with one or more serine or threonine residues (in the case of an O-linked glycosylation site).

The pharmaceutical composition according to one embodiment of the present invention may be applied to all animals, including humans, dogs, chickens, pigs, cows, sheep, guinea pigs, and monkeys.

The pharmaceutical composition according to one embodiment of the present invention may include an additive such as a diluent, an excipient, a lubricant, a binder, a disintegrant, a buffer, a dispersant, a surfactant, a colorant, a flavoring, or a sweetener, if necessary. The pharmaceutical composition according to one embodiment of the present invention may be prepared by a conventional method in the art.

In the present invention, the carrier, excipient, and diluent included in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxybenzoate, talc, magnesium stearate, and mineral water.

The pharmaceutical composition according to one embodiment of the present invention may be administered orally, rectally, transdermally, intravenously, intramuscularly, intraperitoneally, intramedullary, intrathecally, or subcutaneously.

Dosage forms for oral administration may include tablets, pills, soft or hard capsules, granules, powders, solutions, and emulsions, but the present invention is not limited thereto. Dosage forms for parenteral administration may include injections, drops, gels, suspensions, an emulsions, suppositories, patches, and sprays, but the present invention is not limited thereto.

The pharmaceutical composition may be prepared in the form of a sterile injection as an aqueous or oil-based suspension for sterile injection. The suspension may be formulated according to a technique known in the art using an appropriate dispersant or wetting agent (e.g., Tween 80), and an appropriate suspending agent. A sterile injection may be a sterile injectable solution or suspension (e.g., a solution in 1,3-butanediol) in a non-toxic, parenterally acceptable diluent or solvent. Acceptable vehicles and solvents may be mannitol, water, Ringer's solution, and an isotonic sodium chloride solution. In addition, a sterile fixed oil is conventionally used as a solvent or suspending medium. For this purpose, any non-irritating fixed oil, including synthetic mono- or di-glyceride, may also be used. Fatty acids, such as oleic acid and a glyceride derivative thereof, are useful for injections, as are pharmaceutically acceptable natural oil (e.g., olive oil or castor oil) particularly, their polyoxyethylated counterparts.

Parenteral administration of the pharmaceutical composition according to the present invention is particularly useful when desired treatment involves an easily accessible area or organ by topical application. Carriers for topical administration of the composition of the present invention include, but are not limited to, mineral oil, liquid paraffin, white petroleum jelly, propylene glycol, polyoxyethylene, a polyoxypropylene compound, emulsifying wax, and water.

An amount of active ingredient of the pharmaceutical composition according to the present invention may vary according to the age, sex, and weight of a subject to be administered, a pathological condition and its severity, an administration route, and the judgment of a prescriber. An application amount based on these factors may be determined at a level of one of ordinary skill in the art, and a daily dose of the pharmaceutical composition according to the present invention may be, for example, 10 ng/kg/day to 10 mg/kg/day, specifically, 0.1 µg/kg/day to 1 mg/kg/day, more specifically, 1 µg/kg/day to 100 µg/kg/day, and even more specifically, 2 µg/kg/day to 50 µg/kg/day, and may be properly adjusted when there is a difference in effect depending on a dose. The pharmaceutical composition according to one embodiment of the present invention may be administered one to three times a day, but the present invention is not limited thereto.

Terms used herein are intended not to limit the present invention but to describe specific embodiments. Terms without numbers (before a noun) are not intended to their quantity but indicate that there are one or more noun items that are mentioned. The terms "including," "having," and "containing" are interpreted in open terms (that is, meaning as "including but not limited to").

This is because stating a range of values is simply an easy substitute for individually stating each separate value included in the range, and unless explicitly stated otherwise, each separate value is incorporated as if it was individually stated herein. The end values of the range are included in the range, and can be combined independently.

All methods mentioned herein may be performed in any appropriate order unless indicated otherwise or clearly contradicted by the context. The use of any one and all examples or exemplary language (e.g., "such as"), unless included in the claims, is intended only to better describe the present invention, not to limit the scope of the present invention. No language in the specification should be construed as requiring any non-claimed element to be necessary to the implementation of the present invention. Unless defined otherwise, technical and scientific terms used herein have the same meaning as commonly understood by one of ordinary skill in the art to which the present invention belongs.

The present inventors found that treating cancer cells with an anticancer peptide inhibited the proliferation and metastasis of cancer cells and induced apoptosis. Particularly, it was confirmed that apoptosis increases by co-administration of the anticancer peptide of the present invention with an anticancer agent to cancer cells resistant to the anticancer agent.

Accordingly, another aspect of the present invention provides a method of treating cancer, which includes administering the pharmaceutical composition for preventing or treating cancer to a subject in need thereof. The pharmaceutical composition and the administration method thereof are as described above.

Still another aspect of the present invention provides an anticancer adjuvant, which includes a peptide that includes an amino acid sequence selected from the group consisting of SEQ ID NOs: 3 to 5 as an active ingredient.

The content related to the peptide in the anticancer adjuvant is the same as described above, and since the anticancer adjuvant is a type of pharmaceutical composition, the description of the content that overlaps with the pharmaceutical composition for treating cancer described above will be omitted.

The "anticancer adjuvant" used herein refers to a pharmaceutical composition that can aid or increase the activity of an anticancer agent. In the present invention, the anticancer agent may be paclitaxel, docetaxel, abemaciclib, sorafenib, vemurafenib, doxorubicin, gemcitabine, cisplatin, or oxaliplatin, but the present invention is not limited thereto.

In the present invention, the anticancer adjuvant may be administered alone or in combination with an anticancer agent, and in the co-administration, the order of administration is not specifically limited. The anticancer adjuvant may be administered with the anticancer agent simultaneously or sequentially.

According to one embodiment of the present invention, the anticancer adjuvant may be used for anticancer agent-resistant cancer, for example, triple-negative breast cancer.

Yet another aspect of the present invention provides a method of treating cancer, which includes administering the anticancer adjuvant to a subj ect in need thereof. The pharmaceutical composition and the administration method thereof are the same as described above.

### [Advantageous Effects]

A peptide according to one embodiment of the present invention can inhibit the proliferation and metastasis of cancer cells, induce apoptosis, and improve drug resistance, so it can be effectively used for anticancer purposes.

### [Description of Drawings]

FIG. 1 shows the results of evaluating cell viability after treating a triple-negative breast cancer cell line (MDA-MB-231) with anticancer peptides of the present invention (No. 2 to 5) (***p<0.0001 compared with control).
FIG. 2 shows the results of evaluating cell viability after treating normal cells (muscle cells, adipocytes, cardiomyocytes, and nerve cells) with an anticancer peptide (No. 2) (Ns; Not significant).
FIG. 3 shows the results of comparing cell viabilities after treating a triple-negative breast cancer cell line (MDA-MB-231) with an anticancer agent paclitaxel alone or in combination with an anticancer peptide (No. 1 or 2) (* p<0.05, and *** p<0.0001 compared with control).
FIG. 4 shows the results of comparing cell viabilities after treating a triple-negative breast cancer cell line (MDA-MB-231) with an anticancer agent paclitaxel or abemaciclib alone or in combination with an anticancer peptide (No. 2) (* p<0.05, and ** p<0.01 compared with control).
FIG. 5 is a set of cell images (top) confirming the migration of cancer cells and a quantitative graph (bottom) after treating a triple-negative breast cancer cell line (MDA-MB-231) with an anticancer peptide (No. 1 or 2) (* p<0.05, and ** p<0.01 compared with control).
FIG. 6 shows the results of confirming the change in gene expression of MMP-9, which is a marker for the metastasis and migration of cancer cells after treating a triple-negative breast cancer cell line (MDA-MB-231) with an anticancer peptide (No. 1 or 2) (** p<0.01 compared with control).
FIG. 7 shows the results of confirming the change in phosphorylation of Erk protein, which is a marker for drug resistance, viability, and apoptosis inhibition of cancer cells after treating a triple-negative breast cancer cell line (MDA-MB-231) with an anticancer peptide (No. 1 or 2) (* p<0.05 compared with control).
FIG. 8 shows the results of observing the cleavage phenomenon of lamin B 1 protein after treating a triple-negative breast cancer cell line (MDA-MB-231) with an anticancer peptide (No. 1 or 2).
FIG. 9 is a set of graphs of the change in tumor size during the period of experiment (A) and the final tumor size (B) after administering an anticancer peptide (No. 1) to a xenograft tumor animal model in which a triple-negative breast cancer cell line is transplanted into mice (* p<0.05 compared with control).

### [Modes of the Invention]

Hereinafter, one or more specific examples will be described in more detail with reference to examples. However, these examples are provided to illustrate one or more specific examples, and the scope of the present invention is not limited to these examples.

### Peptides

Anticancer peptides used in the present invention are listed in Table 1 below.

**[Table 1]**

| | Peptide sequence | SEQ ID NO: |
|---|---|---|
| No. 1 | EKAEEDRYFR | 1 |
| No. 2 | EQAEEERYFR | 2 |
| No. 3 | YFR | 3 |
| No. 4 | EQAEEER | 4 |
| No. 5 | AQSREQLAALKK | 5 |

### Example 1: In vitro efficacy of inhibiting proliferation and killing cancer cells

To confirm the cytotoxic and anti-proliferative effect of a peptide discovered in the present invention on cancer cells and normal cells, a 3-(4,5-diemthylthiazolyl-2)-2,5-diphenyltetrazolium bromide (MTT) assay was performed. The MTT assay is a method of measuring the growth of living cells and measuring cell viability using MTT reduction by mitochondrial reductase in living cells.

After a cancer cell line (MDA-MB-231) and normal cell lines (L6 skeletal muscle, 3T3-L1 adipocyte, H9C2 cardiomyocyte, and SH-SY5Y neuroblastoma cell lines) were inoculated into 96-well plates and cultured at 37 °C in a 5% CO₂ incubator overnight, the medium was exchanged with a serum-free medium, followed by culturing the cells overnight. Afterward, 1 µM of each peptide (cancer cells: No. 2, 3, 4. and 5; normal cells: No. 2) was treated for 48 hours (a control was treated with the same volume of phosphate-buffered saline (PBS)). After peptide treatment, the plates were washed with PBS twice, and 200 µL of an MTT solution mixed with a medium at 1:40 was added to each well. The plate was wrapped with foil to block light, and stored in an 5% O₂ incubator at 37 °C until the bottom of the plate turned purple. Afterward, the MTT solution was removed, 200 µL of DMSO was added to each well, and the plates were left on a shaker at room temperature for 5 minutes. The solution in each well was transferred to a new plate and absorbance was measured at 570 nm using an ELISA reader. The relative cell proliferation inhibition rate was calculated based on the cell survival rate of the control being set to 100%.

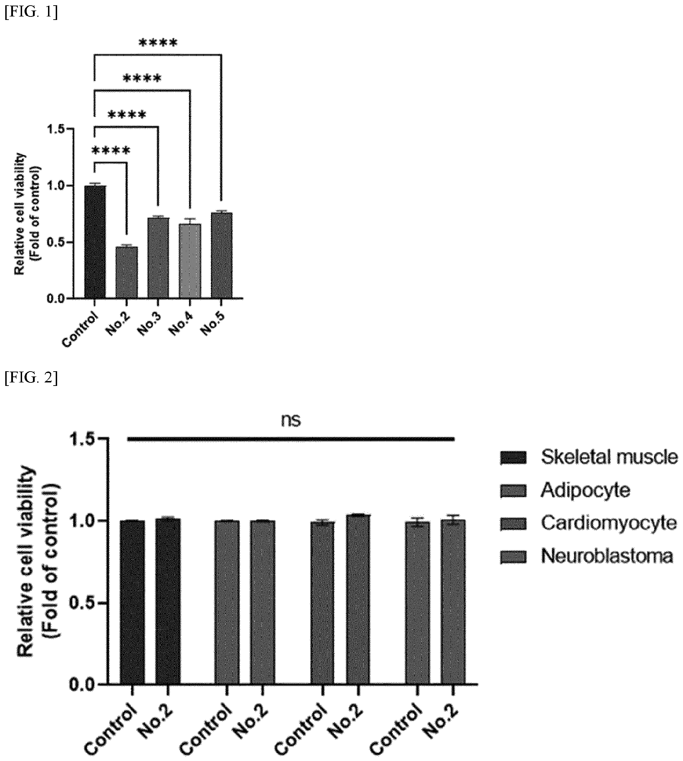

As a result, it was confirmed that the proliferation of cancer cells was significantly reduced by all of the treated peptides (FIG. 1), and there was no change in cell viability of normal cells by the peptides (FIG. 2). These results indicate that the peptides of the present invention are toxic only to cancer cells.

In addition, peptides and anticancer agents were administered together to the MDA-MB-231 triple-negative breast cancer cell line to explore effects associated with drug co-administration and drug resistance. Triple-negative (ER-/PR-/HER2-) breast cancer is a type of breast cancer that is difficult to treat because it is known to have drug resistance to existing hormone therapy and anticancer drugs, and is a suitable model for research on drug resistance and co-administration.

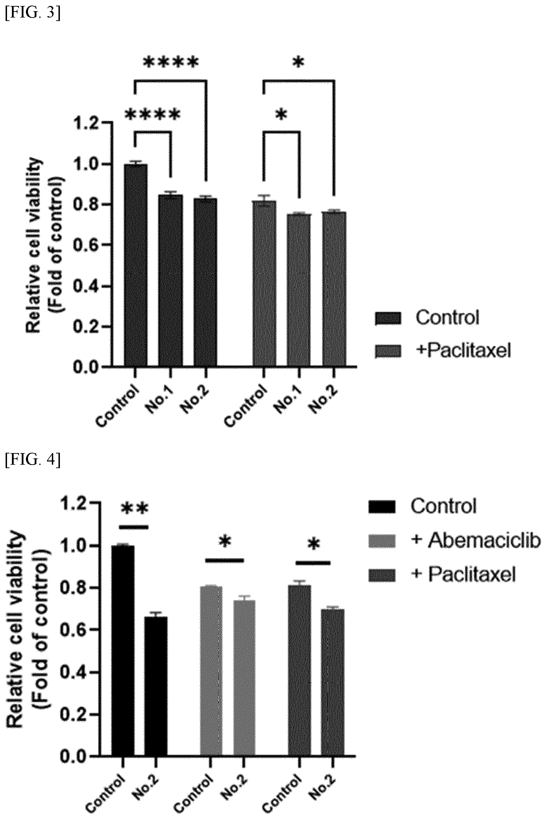

Cell viability was evaluated by an MTT assay by treating MDA-MB-231 cells with 1 µM of the peptide (No. 1 or 2), and 9 µM abemaciclib or 5.4 nM paclitaxel. As a result, it was confirmed that cell proliferation was further inhibited in an experimental group co-treated with the peptide, compared to an experimental group treated with abemaciclib or paclitaxel alone (FIGS. 3 and 4). This result shows the improvement in cancer cell drug resistance and the possibility of co-administration of the anticancer peptide of the present invention with a conventional anticancer agent.

### Example 2: Inhibitory effect on cancer cell migration

In the human body, cancer cells migrate to healthy tissue and cause metastasis through their continuously proliferating characteristics and the creation of microenvironments. Since the metastasis of cancer cells means the transformation of normal tissue into cancerous tissue, the effect of anticancer peptides on the migration of cancer cells was observed through a cell migration experiment. Specifically, MDA-MB-231 cells were cultured in a 6-well plate, each well was scratched in one direction, and a peptide (No. 1 or 2) was treated at a concentration of 1 µM. After 24 hours, a scratch area was observed using an optical microscope.

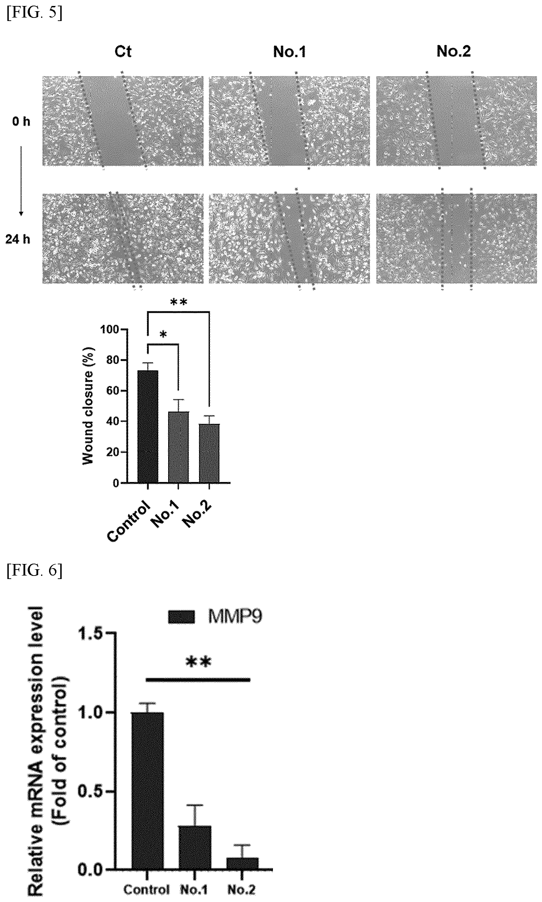

As a result, compared with the control, in the peptide-treated groups, it was confirmed that the number of cells migrating into an empty space is small, resulting in delayed recovery of the scratch area (FIG. 5). This result means that the anticancer peptides can inhibit the migration of cancer cells, and as a result, prevent the metastasis of cancer cells.

### Example 3: Evaluation of action at cell level and apoptosis

The specific intracellular mechanism of action of an anticancer peptide was confirmed as follows.

MDA-MB-231 cells were cultured and treated with 1 µM of a peptide (No. 1 or 2). After 24 hours, RNA was isolated from the cells and the expression level of matrix metallopeptidase 9 (MMP-9), which is a key cell migration factor, was confirmed through RT-PCR. As a result, it was found that the expression of MMP-9 was significantly reduced in the peptide-treated groups compared with the control (FIG. 6).

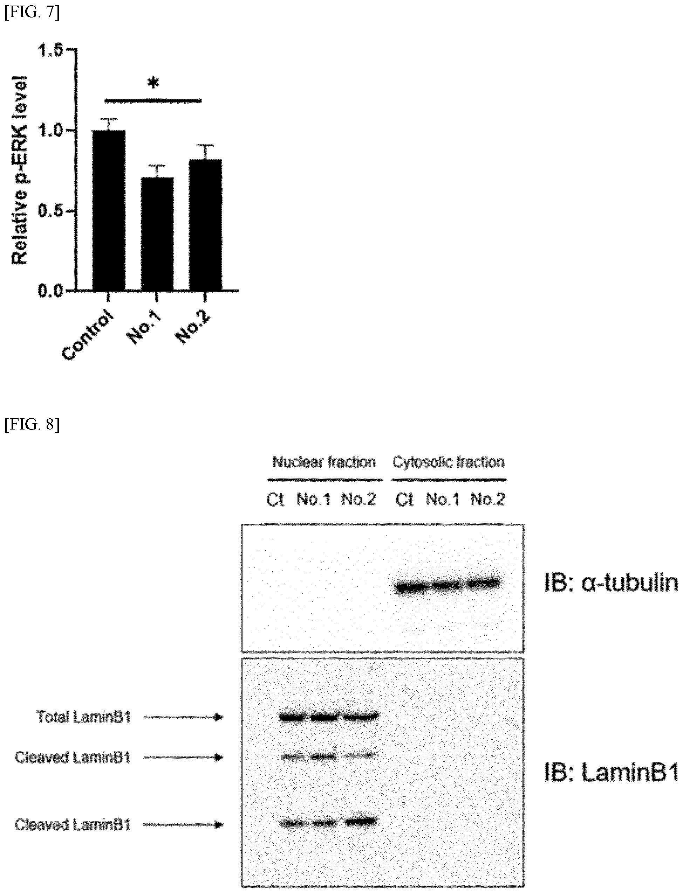

In addition, the change in phosphorylation of extracellular signal-regulated kinase (ERK) protein caused by an anticancer peptide was confirmed. ERK gene is an indicator of drug resistance and inhibition of the growth and apoptosis of cancer cells. The MDA-MB-231 cells were cultured and treated with 1 µM of a peptide (No. 1 or 2). After 24 hours, proteins were isolated from the cells, and a p-ERK level was confirmed by Western blot. As a result, compared with the control, in the peptide-treated groups, it was found that the p-ERK level was significantly reduced (FIG. 7). From this result, it was verified that the drug resistance improvement and cancer cell proliferation inhibition effects of the anticancer peptide target the ERK protein.

Next, to confirm the relationship between the anticancer peptide and apoptosis, the degree of cleavage of lamin B1 protein was confirmed. Lamin B1 is originally a type of nuclear protein that is degraded by caspase in apoptosis and used as a biomarker of apoptosis. The MDA-MB-231 cells were cultured and treated with 1 µM of a peptide (No. 1 or 2). After 24 hours, the cells were washed with PBS twice, and lysed with lysis buffer containing NP-40. The nucleus and cytoplasm were separated by centrifugation, and western blotting was performed on each nuclear and cytoplasmic fraction. Here, to determine the degree of separation of the nucleus and cytoplasm, α-tubulin, which is a cytoplasmic marker, and lamin B1 were simultaneously analyzed. As a result, α-tubulin was not detected in the nuclear fraction, indicating that the nucleus and cytoplasm were successfully separated, and it was confirmed that lamin B 1 was cleaved by the treatment of the anticancer peptides (FIG. 8). This result shows that the anticancer action of the anticancer peptides includes apoptosis.

### Example 4: Evaluation of in vivo anticancer activity

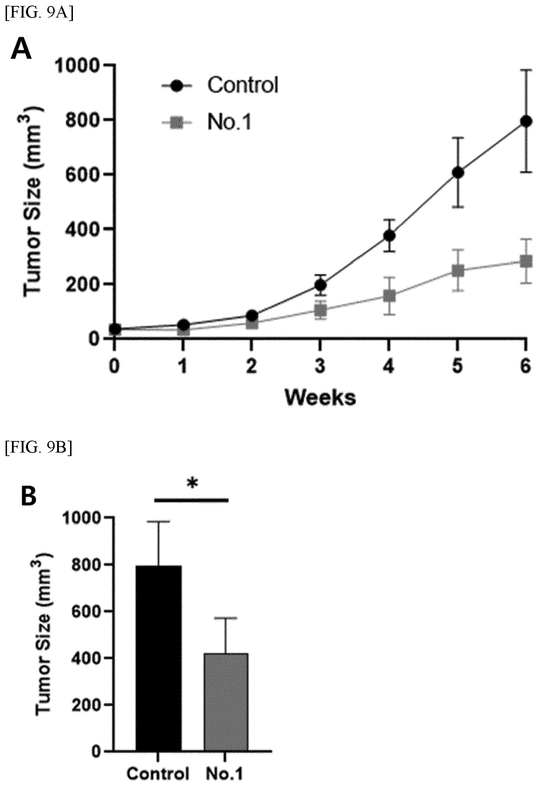

A xenograft tumor model was constructed by injecting MDA-MB-231 cells into immunodeficient BALB/c nude mice and forming tumors for 1 week. Mice with tumors were randomly divided into a control and an experimental group. The control and the experimental group were intraperitoneally injected daily with PBS (the same volume as a peptide) and the No. 1 peptide (2.5 mg/kg), respectively, and then observed for a total of 6 weeks. During the experimental period, a tumor size was measured with a digital caliper every week, and the final tumor size was compared at week 6. As a result, in the peptide-administered group, a significant tumor growth inhibitory effect was confirmed (FIG. 9). This result shows that the anticancer peptide of the present invention acts well in a biological system.

## Claims

1. A pharmaceutical composition for use in preventing or treating cancer, comprising:
a peptide including an amino acid sequence selected from the group consisting of SEQ ID NOs: 3 to 5 as an active ingredient.

2. The pharmaceutical composition for use of claim 1, wherein the peptide including the amino acid sequence represented by SEQ ID NO: 3 is a peptide consisting of an amino acid sequence represented by SEQ ID NO: 1, 2, or 3.

3. The pharmaceutical composition for use of claim 1, wherein the peptide inhibits the metastasis of cancer cells.

4. The pharmaceutical composition for use of claim 1, wherein the peptide induces the apoptosis of cancer cells.

5. A method of treating cancer comprising administering the pharmaceutical composition of claim 1 to a subject in need thereof.

6. An anticancer adjuvant comprising a peptide including an amino acid sequence selected from the group consisting of SEQ ID NOs: 3 to 5 as an active ingredient.

7. The anticancer adjuvant of claim 6, wherein the peptide including the amino acid sequence represented by SEQ ID NO: 3 is a peptide consisting of an amino acid sequence represented by SEQ ID NO: 1, 2, or 3.

8. The anticancer adjuvant of claim 6, wherein the cancer is anticancer agent-resistant cancer.

9. A method of treating cancer comprising administering the anticancer adjuvant of claim 6 to a subject in need thereof.
